# EUROPEAN PATENT APPLICATION

(11) **EP 1 891 917 A1**
(43) Date of publication of application: **27.02.2008**
(21) Application number: 06254438.2
(22) Date of filing: 24.08.2006
(51) Int. Cl.: A61F 11/14, H04R 1/10, B29C 45/44, B29C 44/04

(54) **Cushion cover for earmuffs**

(71) Applicant: Unique Safety Equipment Co., Ltd., Taipei (TW)
(72) Inventor: Chen, Ling-Go, Taipei (TW)
(74) Representative: Skone James, Robert Edmund

(57) **Abstract**

A cushion cover for use with earflaps is proposed, comprising: a soundproof cushion having a first surface and a second surface; a covering layer having an outer surface and an inner surface, the inner surface of the covering layer being completely adhered to the first surface of the soundproof cushion; and a rubber casing having an outer casing surface and an inner casing surface, the inner casing surface of the rubber casing being completely adhered to the second surface of the soundproof cushion, thereby eliminating the necessity of two separate binding processes, and improving durability while reducing the production cost.

## Description

### FIELD OF INVENTION

The present invention relates to a cushion cover for earflaps, and more particularly, to a cushion cover designed for use with earflaps having a soundproof cushion for reducing external noise.

### BACKGROUND ART

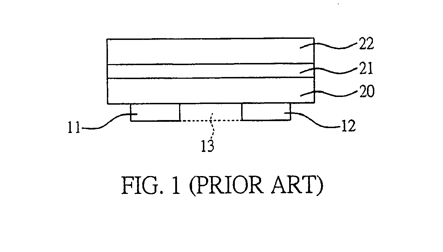

A general earflap-type earphone used for listening to music and to block high-decibel noise at a workplace, or to reduce or cut off external noise is basically composed of a cushion cover that is provided with an adjustable connection frame having two ends thereof fastened to cover the wearer's ears. The cushion cover typically constitutes, as shown in FIG. 1, a soundproof cushion 10 that is made of a foam sponge material and enclosed by a soft-resin surface layer 11 made of a high molecular material. A conventional method is to use an adhesive 12 (or a high cycle wave bonding method) to securely enclose the soundproof cushion in the soft-resin surface layer 11. Thereafter, the soundproof cushion 10 enclosed by the surface layer 11 is adhered to a rubber casing 14 to form the cushion cover 1 as shown in FIG. 1.

The aforementioned cushion cover 1 basically comprises the soundproof cushion 10, the soft-resin surface layer 11, and the rubber casing 14. This design has inherent disadvantages in that the three major constituent components mentioned above require independent manufacture using two separate binding processes, adversely increasing manufacturing costs. First, the soft-resin surface layer 11 has to be adhered to the soundproof cushion 10 by means of adhesives or other known methods; next, the soundproof cushion 10 is to be enclosed by the soft-resin surface layer 11, subsequently, the soundproof cushion 10 enclosed with the soft-resin surface layer 11 has to be adhered to the rubber casing 14 by a conventional adhering medium such as double-sided adhesive or adhesive. Such a manufacturing process requiring two separate adhering processes is laborious and time-consuming, and binding methods like conventional adhesives, double-sided adhesives, or high cycle waves all adversely increase costs and thus fail to satisfy the desire for low-cost manufacturing.

Further, the soft-resin surface layer 11 is made of a high molecular material in a big sheet and then is cut into small pieces for practical application, yet the cutting process inevitably generates waste materials and increases the cost as well.

Moreover, in order to lower the cost, the binding between the soft-resin surface layer I 1 and the soundproof cushion 10, and between the soundproof cushion 10 and the rubber casing 14 are only partially done and, thus, such elements are not completely adhered to one another. This method is prone to defects as the soft-resin surface layer 11 could easily come off from the soundproof cushion 10 or the soundproof cushion may easily detach from the rubber casing 14 that lead to problems of a short lifespan of the finished products. Also, there have been other disadvantages arising from the use of conventional covers for earphones. Such conventional covers have been inferior due to inherent characteristics and deformation of the materials from which the covers have been made.

### DISCLOSURE OF UTILITY MODEL

It is therefore an objective of the present invention to provide a novel cushion cover for use with earflaps that simplifies the manufacturing process and reduces costs.

Another objective of the present invention is to provide a novel cushion cover for use with earflaps that can enhance durability and prolong the lifespan of the products.

Still another objective of the present invention is to provide a novel cushion cover for use with earflaps that does not require use of any adhering medium or conventional methods so that the manufacturing cost can be effectively reduced.

To achieve the above and other objectives, the present invention proposes a novel cushion cover for use with earflaps, comprising: a soundproof cushion having a first surface and a second surface; a covering layer having an outer surface and an inner surface, the inner surface of the covering layer being completely adhered to the first surface of the soundproof cushion by means of the inherent adherence of the soundproof cushion; and a rubber casing having an outer casing surface and an inner casing surface, the inner casing surface of the rubber casing being completely adhered to the second surface of the soundproof cushion by means of the inherent adherence of the soundproof cushion.

The material used for making the soundproof cushion, the covering layer, and the rubber casing of the present invention can be any suitable materials known in the art without specific limitation, so long as the foam material used for making the soundproof cushion can provide sufficient cohesion to fully bind the covering layer together with the rubber casing after a foaming process.

In summary, the binding of the soundproof cushion with the covering layer and the soundproof cushion with the rubber cushion is fully intact without requiring any use of an adhering medium or methods, thereby effectively simplifying the manufacturing process and lowering the cost while enhancing durability of the fabricated products due to the strong binding between the soundproof cushion, the covering layer, and the rubber casing respectively.

### BRIEF DESCRIPTION OF DRAWINGS

The foregoing and other objects, aspects, and advantages will be better understood from the following detailed description of the invention with reference to the drawings, in which:
FIG. 1 (PRIOR ART) is a cross-sectional view of a conventional cushion cover fitted on an earflap;
FIG. 2 is a perspective view of the cushion cover according to the invention;
FIGS. 3A to 3D together comprise a flow diagram showing the steps of manufacturing the cushion cover for use with earflaps according to the present invention.

### BEST MODE FOR CARRYING OUT THE INVENTION

The present invention is described in the following so that one skilled in the pertinent art can easily understand other advantages and effects of the present invention. The present invention may also be implemented and applied according to other embodiments, and the details may be modified based on different views and applications without departing from the spirit of the invention.

For the sake of simplicity and conciseness, the other components required for applying the cushion cover for earflaps, such as an amplifier, a power wire or a receiver are purposely omitted from the description as those elements are well known in the art.

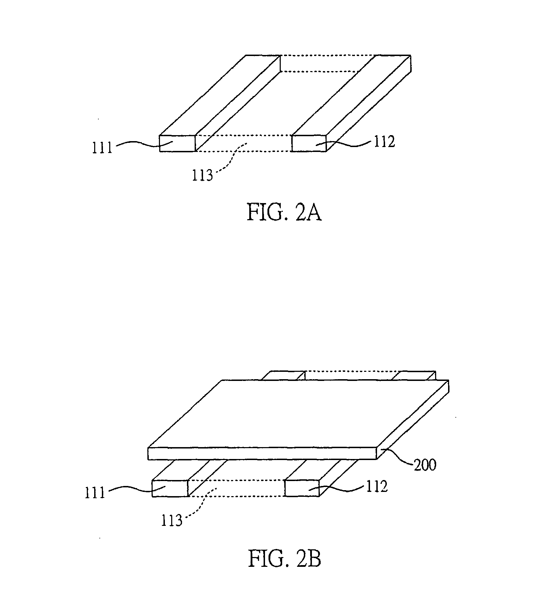

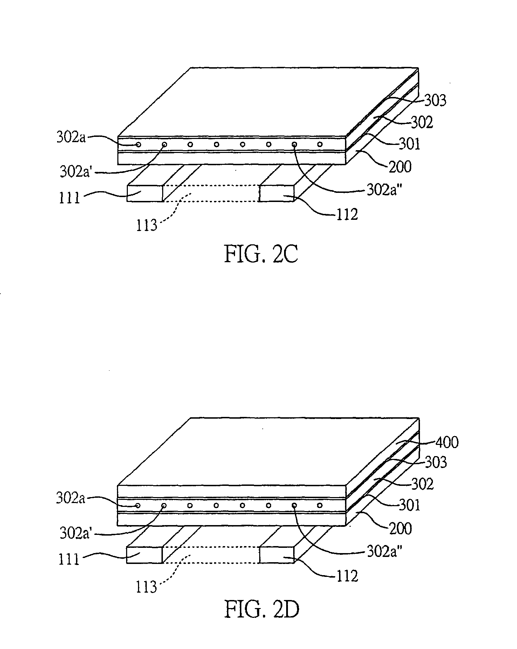

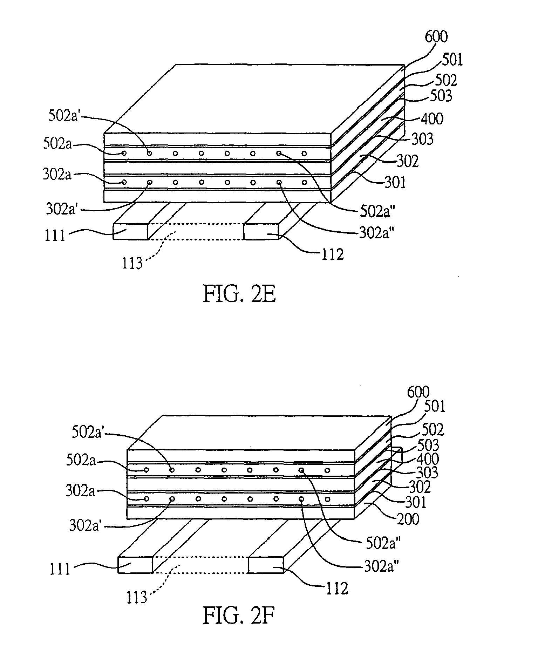

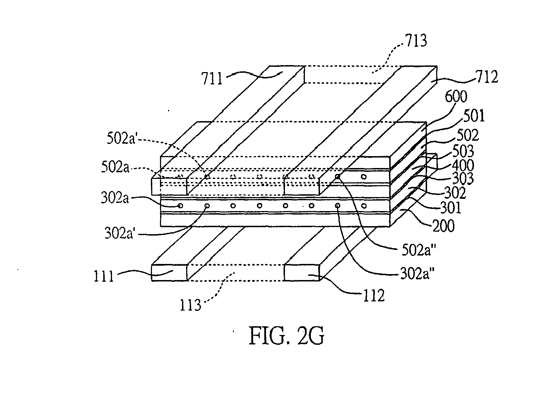

Referring to FIG. 2, a cushion cover 2 is shown for use with earflaps that is composed of a covering layer 20, a soundproof cushion 21, and a rubber casing 22. The covering layer 20 has an outer surface 200 and an inner surface 201 corresponding to the outer surface 200, and the soundproof cushion 21 has a first surface 210 facing the covering layer 20 and a second surface 211 facing the rubber casing 22. The inner surface 201 of the covering layer 20 is completely adhered to the first surface 210 of the soundproof cushion 21 by means of the inherent adhering characteristics of the soundproof cushion 21, such that the covering layer is smoothly adhered to the soundproof cushion 21 in such a way that improves the appearance of the fabricated cushion cover 2 and provides a smoother touch. The rubber casing 22 also has an outer casing surface 220 exposed at the ear side of the earphone and an inward-facing casing surface 221 towards the interior of the earphone that permits the inner casing surface 221 of the rubber casing 22 to be completely adhered to the second surface 211 of the soundproof cushion 21 by means of the inherent adhering characteristics of the soundproof cushion 21.

The covering layer 20, the soundproof cushion 21, and the rubber casing 22 are made of high molecular materials, which are well-known in the art and thus will not be further described herein for brevity. However, it is to be noted that in this embodiment the foam material used for making the soundproof cushion 21 must have sufficient cohesion necessary for fixedly binding the covering layer 20 and the rubber casing 22. By the inherent adhering characteristics of the soundproof cushion 21, the covering layer 20 and the rubber casing 22 can be bound effectively without using adhesives, two-way adhesives, or a high cycle wave method for binding the covering layer 20 and the rubber casing 22, thereby saving costs and simplifying the manufacturing process while increasing production efficiency.

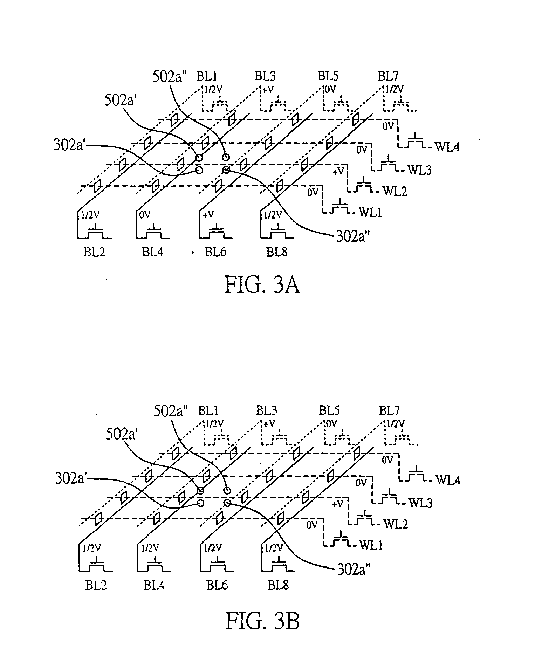

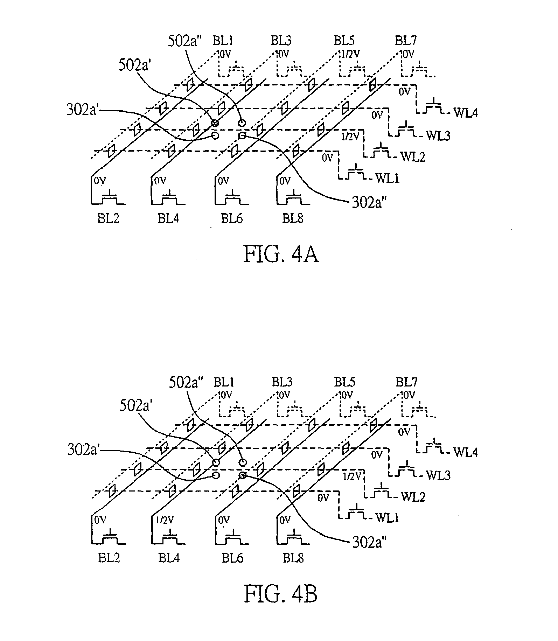

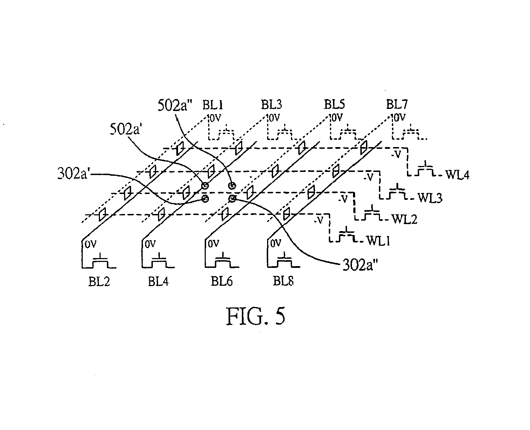

FIG. 3 is a schematic flow diagram showing the steps of manufacturing the cushion cover 2 for use with earflaps according to the present invention. As shown in FIG. 3A, the first step comprises coating the surface of a mold cavity 230 of a mold 23 with a covering layer 20. Suitable material for making the covering layer 20 has a cohesion degree between the covering layer 20 and the surface of the mold cavity 230 of the mold 23 smaller than that between the covering layer 20 and the soundproof cushion 21, such that after the production process, the covering layer 20 is completely adhered to the soundproof cushion 21 when the mold 23 is detached from the fabricated products.

As shown in FIG 3B, the outer casing surface 220 of the rubber casing 22 and the inner casing surface 221 corresponding to the outer casing surface 220 are combined on the mold 23 to perform the subsequent molding process. After the rubber casing 22 is covered on the mold 23, the inner casing surface 221 faces toward the mold cavity 230, and the rubber casing 22 is formed with a plurality of injection apertures 222 for connecting the mold cavity 230 to the outside. Further, the circumference of the rubber casing 22 is formed with a stairway portion 223 which is adapted to closely seal the circumference of the mold cavity 230 via the stairway portion 223, such that the foam material being injected into the mold cavity 230 is prevented from overflowing from the circumference of the mold cavity 230 in the subsequent molding process.

Thereafter, as shown in FIG. 3C, a molding process is performed to inject the foam material via the injection apertures 222 of the rubber casing 22 into the mold cavity 230, allowing the foam material to foam in the mold cavity 230 to form the soundproof cushion 21. As the foam material used is inherent with a cohesive property, the fabricated soundproof cushion 21 has the cohesion required to enable the inner surface 201 of the covering layer 20 to be fully bound with the first surface 210 of the soundproof cushion 21, while the inner casing surface 221 of the rubber casing 22 is completely bound with the second surface 211 of the soundproof cushion 21. The method achieves not only the purpose of utilizing the molding process to form the soundproof cushion 21 but also the effect of binding the covering layer 20, the soundproof cushion 21, and the rubber casing 22 with one another effectively, thereby eliminating the two steps of binding required in conventional cover structures, simplifying the manufacturing process and reducing costs as a result. Moreover, the covering layer 20 and the rubber casing 22 can be fully adhered to the first surface 210 and the second surface 211 of the soundproof cushion 21. Consequently, the cohesive bonds between the covering layer 20 and the soundproof cushion 21, and the cohesion between the rubber casing 22 and the soundproof cushion 21 are far greater than in conventional covers and can effectively increase durability and lifespan of the fabricated products.

Lastly, as shown in FIG. 3D, the mold 23 is detached from the cushion cover 2 consisting of the covering layer 20, the soundproof cushion 21, and the rubber casing 22 in the direction shown by an arrow in the drawing. As mentioned earlier, the chosen material for making the covering layer 20 has cohesion with the inner surface of the mold cavity 230 smaller than that with the soundproof cushion 21, such that the covering layer 20 will not strongly adhere to the surface of the mold cavity 230 and prevent smooth detachment of the mold 23 from the cushion cover 2. Certainly, a detachment layer can be used to coat the surface of the mold cavity 230 to ensure that the covering layer 20 does not adhere to the mold 23 to facilitate the off-mold process.

It is to be noted that the manufacturing method of the present invention is not limited to the disclosure of the foregoing embodiment. For instance, the soundproof cushion 21 may be formed first and then the covering layer 20 can be formed on the first surface 210 of the soundproof cushion 21 to simplify the process and reduce the cost. Further, as the soundproof cushion of the present invention is made of foam materials, the flexibility of the soundproof cushion can be suitably adjusted, and also the color, the size, or even the shape thereof can be chosen as desired and made to order to meet practical requirements. Also, the foam material used for making the soundproof cushion can be one that is environmentally-friendly that does not generate toxic and harmful substances during burning processes.

Having thus described a preferred embodiment in sufficient detail to enable those skilled in the art to make and use the invention, it will nevertheless be appreciated that numerous variations and modifications of the illustrated embodiment may be made without departing from the spirit of the invention, and it is intended that the invention not be limited by the above description or accompanying drawings, but that it be defined solely in accordance with the appended claims.

## Claims

1. A cushion cover for use with earflaps, comprising:
a covering layer having an outer surface and an inner surface;
a rubber casing having an outer, earward-facing casing surface and an inward-facing casing surface; and
a soundproof cushion having a first surface facing the covering layer and a second surface facing the rubber casing, the inner surface of the covering layer being completely adhered to the first surface of the soundproof cushion by means of the inherent adhering characteristics of the soundproof cushion, and the inner casing surface of the rubber casing being completely adhered to the second surface of the soundproof cushion by means of the inherent adhering characteristics of the soundproof cushion, thereby surrounding the soundproof cushion within a space covered and defined by the covering layer and the rubber casing.

2. The cushion cover according to claim 1, wherein the circumference of the rubber casing is further formed with a stairway portion.

3. The cushion cover according to claim 1 or claim 2, wherein the soundproof cushion is made of a foam material by a foaming process.

4. The cushion cover according to claim 3, wherein the foam material has an adhering property that allows the rubber casing to be effectively bound with the covering layer after the soundproof cushion is formed by a foaming process.

5. The cushion cover according to claim 3 or claim 4, wherein the rubber casing is further formed with a plurality of injection apertures for allowing the foam material to be injected into the space surrounded and defined by the covering layer and the rubber casing to thereby foam therein and form the soundproof cushion.
